# EUROPÄISCHE PATENTSCHRIFT

(11) **EP 2 917 740 B1**
(45) Veröffentlichungstag und Bekanntmachung des Hinweises auf die Patenterteilung: **04.01.2017**
(21) Anmeldenummer: 13786487.2
(22) Anmeldetag: 06.11.2013
(51) Int. Cl.: G01N 33/84, C07K 7/00

(54) **IN BESCHICHTUNGSMITTELN, HAFTVERMITTLERN ODER KLEBSTOFFEN FÜR OXIDISCHE OBERFLÄCHEN EINSETZBARES PEPTID**
PEPTIDE THAT CAN BE USED IN COATING AGENTS, ADHESION PROMOTERS OR ADHESIVES FOR OXIDIC SURFACES
PEPTIDE UTILISABLE DANS DES AGENTS DE REVÊTEMENT, DES PROMOTEURS D'ADHÉSION OU DES COLLES POUR SURFACES OXYDANTES

(30) Priorität: 07.11.2012 DE 102012110664
(43) Veröffentlichungstag der Anmeldung: 16.09.2015
(60) Teilanmeldung: 16183798.4
(73) Patentinhaber: Henkel AG & Co. KGaA, 40589 Düsseldorf (DE)
(72) Erfinder: TADEN, Andreas, 40597 Düsseldorf (DE); VEITH, Birgit, 40235 Düsseldorf (DE); BREVES, Roland, 40822 Mettmann (DE); SCHMIDT, Irmgard, 42699 Solingen (DE); WEBER, Thomas, 41541 Dormagen (DE); JOSE, Joachim, 40589 Düsseldorf (DE); REICHENEDER, Claudia, 52249 Eschweiler (DE)
(86) Internationale Anmeldenummer: PCT/EP2013/073109
(87) Internationale Veröffentlichungsnummer: WO 2014/072313

(56) Entgegenhaltungen:
- WO-A2-03/078451
- WO-A2-2009/079053
- WO-A2-2010/000493
- JP-A- 2006 225 294
- BANEYX ET AL: "Selection and analysis of solid-binding peptides", CURRENT OPINION IN BIOTECHNOLOGY, LONDON, GB, Bd. 18, Nr. 4, 14. September 2007 (2007-09-14), Seiten 312-317, XP022244964, ISSN: 0958-1669, DOI: 10.1016/J.COPBIO.2007.04.008
- SARIKAYA ET AL: "materials assembly and formation using engineered polypeptides", ANNUAL REVIEW OF MATERIALS RESEARCH, ANNUAL REVIEWS, PALO ALTO, CA, US, Bd. 34, 1. Januar 2004 (2004-01-01), Seiten 373-408, XP009125941, ISSN: 1531-7331
- BRADFORD ET AL: "A rapid and sensitive method for the quantitation of microgram quantities of protein utilizing the principle of protein-dye binding", ANALYTICAL BIOCHEMISTRY, ACADEMIC PRESS INC, NEW YORK, Bd. 72, Nr. 1-2, 7. Mai 1976 (1976-05-07), Seiten 248-254, XP025650297, ISSN: 0003-2697, DOI: 10.1016/0003-2697(76)90527-3 [gefunden am 1976-05-07]
- KAISER E ET AL: "Color test for detection of free terminal amino groups in the solid-phase synthesis of peptides", ANALYTICAL BIOCHEMISTRY, ACADEMIC PRESS INC, NEW YORK, Bd. 34, Nr. 2, 1. April 1970 (1970-04-01), Seiten 595-598, XP024828956, ISSN: 0003-2697, DOI: 10.1016/0003-2697(70)90146-6 [gefunden am 1970-04-01]
- CORRINE K THAI ET AL: "Identification and Characterization of Cu20- and ZnO-Binding Polypeptides by Escherichia coli Cell Surface Display:Toward an Understanding of Metal Oxide Binding", BIOTECHNOLOGY AND BIOENGINEERING, WILEY & SONS, HOBOKEN, NJ, US, Bd. 87, Nr. 2, 20. Juli 2004 (2004-07-20), Seiten 129-137, XP008156557, ISSN: 0006-3592, DOI: 10.1002/BIT.20149 [gefunden am 2004-06-22]

### SEQUENCE LISTING

<110> Henkel AG & Co. KGaA,
<120> In Beschichtungsmitteln, Haftvermittlern oder Klebstoffen für
   oxidische Oberflächen einsetzbare Peptide
<130> HenkAdP-01
<141> 2012-11-07
<160> 19
<170> SeqWin99, version 1.02
<210> 1
   <211> 36
   <212> DNA
   <213> Artificial Sequence
<220>
   <223> Artificial Sequence
<220>
   <221> Für Peptid 6 kodierende Nukleinsäure
<400> 1
   tccagggcac gactatttgt agtcacatat cacaaa
   36
<210> 2
   <211> 36
   <212> DNA
   <213> Artificial Sequence
<220>
   <223> Artificial Sequence
<220>
   <221> Für Peptid 7 kodierende Nukleinsäure
<400> 2
   cacatgatat ctacaatgaa cgcggcgagc cggcga
   36
<210> 3
   <211> 36
   <212> DNA
   <213> Artificial Sequence
<220>
   <223> Artificial Sequence
<220>
   <221> Für Peptid 8 kodierende Nukleinsäure
<400> 3
   agaagcatag taacctttag tttaagacaa aatcgc
   36
<210> 4
   <211> 36
   <212> DNA
   <213> Artificial Sequence
<220>
   <223> Artificial Sequence
<220>
   <221> Für Peptid 9 kodierende Nukleinsäure
<400> 4
   caacgtaatt tgattaaatc gacgtgccgg aaaatc
   36
<210> 5
   <211> 36
   <212> DNA
   <213> Artificial Sequence
<220>
   <223> Artificial Sequence
<220>
   <221> Für Peptid 10 kodierende Nukleinsäure
<400> 5
   cgccagctcc agaggctgat gaaatcagta aattcg
   36
<210> 6
   <211> 36
   <212> DNA
   <213> Artificial Sequence
<220>
   <223> Artificial Sequence
<220>
   <221> Für Peptid 13 kodierende Nukleinsäure
<400> 6
   cgcaacacta ttcgtatacg gactataaaa catccg
   36
<210> 7
   <211> 36
   <212> DNA
   <213> Artificial Sequence
<220>
   <223> Artificial Sequence
<220>
   <221> Für Peptid 15 kodierende Nukleinsäure
<400> 7
   cgacacagta gtactctgag gtataggcca cttccg
   36
<210> 8
   <211> 36
   <212> DNA
   <213> Artificial Sequence
<220>
   <223> Artificial Sequence
<220>
   <221> Für Peptid 17 kodierende Nukleinsäure
<400> 8
   caacaaagtc gtcatatact gaatagaaaa aaaccg
   36
<210> 9
   <211> 12
   <212> PRT
   <213> Artificial Sequence
<220>
   <223> Artificial Sequence
<220>
   <221> Peptid 6
<400> 9
   Ser Arg Ala Arg Leu Phe Val Val Thr Tyr His Lys
   1 5 10
<210> 10
   <211> 12
   <212> PRT
   <213> Artificial Sequence
<220>
   <223> Artificial Sequence
<220>
   <221> Peptid 7
<400> 10
   His Met Ile Ser Thr Met Asn Ala Ala Ser Arg Arg
   1 5 10
<210> 11
   <211> 12
   <212> PRT
   <213> Artificial Sequence
<220>
   <223> Artificial Sequence
<220>
   <221> Peptid 8
<400> 11
   Arg Ser Ile Val Thr Phe Ser Leu Arg Gln Asn Arg
   1 5 10
<210> 12
   <211> 12
   <212> PRT
   <213> Artificial Sequence
<220>
   <223> Artificial Sequence
<220>
   <221> Peptid 9
<400> 12
   Gln Arg Asn Leu Ile Lys Ser Thr Cys Arg Lys Ile
   1 5 10
<210> 13
   <211> 12
   <212> PRT
   <213> Artificial Sequence
<220>
   <223> Artificial Sequence
<220>
   <221> Peptid 10
<400> 13
   Arg Gln Leu Gln Arg Leu Met Lys Ser Val Asn Ser
   1 5 10
<210> 14
   <211> 12
   <212> PRT
   <213> Artificial Sequence
<220>
   <223> Artificial Sequence
<220>
   <221> Peptid 13
<400> 14
   Arg Asn Thr Ile Arg Ile Arg Thr Ile Lys His Pro
   1 5 10
<210> 15
   <211> 12
   <212> PRT
   <213> Artificial Sequence
<220>
   <223> Artificial Sequence
<220>
   <221> Peptid 15
<400> 15
   Arg His Ser Ser Thr Leu Arg Tyr Arg Pro Leu Pro
   1 5 10
<210> 16
   <211> 12
   <212> PRT
   <213> Artificial Sequence
<220>
   <223> Artificial Sequence
<220>
   <221> Peptid 17
<400> 16
   Gln Gln Ser Arg His Ile Leu Asn Arg Lys Lys Pro
   1               5                   10
<210> 17
   <211> 12
   <212> PRT
   <213> Artificial Sequence
<220>
   <223> Artificial Sequence
<220>
   <221> Peptid K1
<400> 17
   Xaa Lys Arg Arg Pro Gly Xaa Xaa Val Glu Xaa Xaa
   1               5                   10
<210> 18
   <211> 12
   <212> PRT
   <213> Artificial Sequence
<220>
   <223> Artificial Sequence
<220>
   <221> Peptid K2
<400> 18
   His Asn Thr Ile Arg Ile Leu Thr Ile Lys Leu Xaa
   1               5                   10
<210> 19
   <211> 12
   <212> PRT
   <213> Artificial Sequence
<220>
   <223> Artificial Sequence
<220>
   <221> Peptid K3
<400> 19
   Arg His Ser Ser Thr Leu Arg Tyr Arg Pro His Pro
   1               5                   10

## Beschreibung

Die Erfindung betrifft Peptide, insbesondere Dodekapeptide enthaltende Beschichtungsmittel, Haftvermittler oder Klebstoffe für oxidische Oberflächen, einen mehrschichtigen Verbund oder ein beschichtetes Substrat, enthaltend Verbindungen, die vollständig oder zum Teil aus Dodekapeptiden als Haftvermittler zwischen mindestens zwei benachbarten Schichten des Verbunds oder zwischen der Beschichtung und dem Substrat aufgebaut sind sowie als Beschichtungsmittel, Haftvermittler oder Klebstoffe für oxidische Oberflächen einsetzbare Dodekapeptide.

Im Bereich der Beschichtung oder Lackierung oxidischer Oberflächen sind bekannte Methoden zur Haftvermittlung zum Beispiel bifunktionelle Organosilanverbindungen, die eine chemische Bindung zwischen der Polymermatrix des Lackes und dem anorganischen Substrat herstellen. Ein Nachteil dieser Haftvermittler besteht unter anderem darin, dass eine optimale Haftung nur gewährleistet ist, wenn sich auf der Oberfläche des Substrates Hydroxid-Gruppen befinden, mit denen das Silan wechselwirken kann. In vielen Fällen ist zur Silanisierung von Oberflächen daher eine aufwendige Vorbehandlung des Substrates notwendig. Auch die Herstellung von maßgeschneiderten Materialien ist sehr aufwendig und zudem sind nicht für alle Substrate maßgeschneiderten Materialien bekannt.

Self-Assembled Monolayers (auch Selbstorganisierende Monoschichten, Abkürzung: SAM's) wurden schon 1940 untersucht und beschrieben, aber erst 1983 hat man angefangen, diesen Ordnungsprozess technisch zu nutzen.

Self-Assembled Monolayers haben im Prinzip zwei unterschiedliche aktive Gruppen, dazwischen ist ein Spacer, z. B. aliphatischer Spacer. Eine Gruppe davon wechselwirkt mit der Oberfläche und die andere Endgruppe wirkt mit einer speziellen Eigenschaft oder verbindet sich mit einer anderen funktionellen Verbindung. Die SAMs bilden chemische Bindungen aus, die dem Angriff von Wasser widerstehen und gleichzeitig alle elektrochemischen Reaktionen an den Zwischenschichten verhindern können. Die Selbstorganisation der monomolekularen Schichten auf fester Oberfläche bietet ein rationelles Verfahren für die Herstellung einer Zwischenschicht mit einer definierten Zusammensetzung, Struktur und Dicke. Selektiv selbstorganisierende Haftvermittler haben den Vorteil, die Haftung der Lack- und Klebstoffschichten an metallischen Oberflächen für lange Lebenszeiten zu gewährleisten, wie es zum Beispiel im Automobilbereich für eine weitere Verbesserung des Korrosionsschutzes erforderlich ist. Sie ermöglichen weiterhin eine gleichmäßig gute Haftung einer einheitlichen Lackschicht auf den vermehrt zum Einsatz kommenden Kompositwerkstoffen mit unterschiedlichen Oberflächen wie Kunststoff-Metallverbundwerkstoffe.

Selbstorganisierende Monoschichten werden beispielsweise in der Halbleitertechnologie zur Oberflächenstabilisierung und maßgeschneiderten Funktionalisierung von Elektroden eingesetzt. Je nach Länge der verwendeten Alkylketten wird dabei die Permeabilität und die Ladungstransfergeschwindigkeit beeinflußt. Das Anwendungsgebiet selbstorganisierender Monoschichten ist sehr breit. Unter anderem wird die Technik der selbstorganisierenden Monoschicht beim elektrochemischen Rastertunnelmikroskop, bei Zelluntersuchungen, bei der Sensorik und in der Nanoelektronik verwendet. Allerdings ist die Erzeugung der im Stand der Technik beschriebenen SAMs aufwendig und kostspielig.

Die Kopplung eines anorganischen Substrats mit biologischen Komponenten zur Modifikation der Oberflächeneigenschaften ist in der Biomimetik bekannt. Aus einer Phagenbibliothek selektierte, kurze Peptide präsentierende Bakteriophagen wurden beispielsweise dazu verwendet, anorganische Materialien zu fällen bzw. abzuscheiden (WO2003/078451). Auch Hybridmaterialien aus einem anorganischen Substrat und spezifischen Peptid-Liganden werden als potentielle Lösung zur Veränderung der Substratoberfläche verwendet. Die Identifikation des an das Substrat passenden biologischen Liganden (üblicherweise ein Peptid) ist gängige Praxis. So sind z.B. eine Vielzahl von Dodekapeptiden bekannt, die auf oxidischen Oberflächen haften (Baneyx et al, CURRENT OPINION IN BIOTECHNOLOGY, 18(4), 2007, Seiten 312-317; Sarikaya et al, ANNUAL REV MATER RES, 34, 2004, 373-408; WO2003/078451; WO2009/079053; JP2006/225294; WO2010/000493; Thai et al, BIOTECHNOL BIOENG, 87(2), 2004, 129-137). Die Identifikation ist aber zeit- und kostspielig und stand bisher einer konkreten Anwendung im Wege. Das Konzept eines bifunktionellen Liganden zur Bindung von zwei anorganischen Komponenten wird im Stand der Technik angesprochen. Konkret wurde die Bindung von Zellen oder Biomolekülen an einem Polymersubstrat, insbesondere an oxidiertem Chlor-angereicherten Polypyrrol (PPyCl) beziehungsweise Poly(lactatco-glycolat) (PLGA), durch Bakteriophagen mit bifunktionellen Bindungseigenschaften zum Beispiel in WO2004/035612 beschrieben. Die weitere Bindung an einen weiteren Stoff, der wie zum Beispiel ein Beschichtungsmaterial, kein biologischer Bindungspartner des verwendeten Phagen ist, wird nicht weiter beschrieben, wobei diese Bindung ebenfalls selektiv zu erfolgen hätte und eine genaue Anpassung des Liganden an die weitere Bindungskomponente erfordern würde.

Es besteht daher ein erheblicher Bedarf an der Bereitstellung neuer, für den Einsatz auf oxidischen Oberflächen geeigneter Verbindungen, die die genannten Nachteile des Standes der Technik vermeiden.

Überraschenderweise wurde gefunden, daß bestimmte Dodekapeptide vorteilhaft als Beschichtungsmittel, Haftvermittler oder Klebstoffe für oxidische Oberflächen eingesetzt werden können.

Ein Gegenstand der vorliegenden Erfindung ist daher ein Dodekapeptid mit der in Tabelle 2 als Peptid 7 aufgeführten Aminosäuresequenz.

Weitere Gegenstände der vorliegenden Erfindung sind für das erfindungsgemäße Peptid kodierende Nukleinsäuren, insbesondere die in der Tabelle 3 aufgeführte Nukleinsäuresequenz.

Unter einem Peptid ist im Sinne der vorliegenden Anmeldung ein aus den natürlichen Aminosäuren zusammengesetztes, weitgehend linear aufgebautes Polymer zu verstehen, das kleiner ist als ein natürliches Protein. In der vorliegenden Anmeldung werden die 19 proteinogenen, natürlich vorkommenden L-Aminosäuren mit den international gebräuchlichen 1-und 3-Buchstaben-Codes bezeichnet.

Das erfindungsgemäße Dodekapeptid lässt sich chemisch durch bekannte Verfahren der Peptidsynthese, beispielsweise durch Festphasensynthese nach Merrifield herstellen.

Bevorzugt ist es jedoch, das erfindungsgemäße Peptid mittels rekombinanter Verfahren herzustellen. Hierunter sind erfindungsgemäß alle gentechnischen oder mikrobiologischen Verfahren zu verstehen, die darauf beruhen, daß die Gene für die interessierenden Peptide in einen für die Produktion geeigneten Wirtsorganismus eingebracht und von diesem transkribiert und translatiert werden. Geeigneterweise erfolgt die Einschleusung der betreffenden Gene über Vektoren, insbesondere Expressionsvektoren; aber auch über solche, die bewirken, daß das interessierende Gen im Wirtsorganismus in ein bereits vorhandenes genetisches Element wie das Chromosom oder andere Vektoren eingefügt werden kann. Die funktionelle Einheit aus Gen und Promotor und eventuellen weiteren gegentischen Elementen wird erfindungsgemäß als Expressionskassette bezeichnet. Sie muß dafür jedoch nicht notwendigerweise auch als physische Einheit vorliegen.

Besonders bevorzugtermaßen wird das erfindungsgemäße Peptid als Multimer hergestellt und nachfolgend in die funktionalen Peptide gespalten. Ganz besonders bevorzugte Multimere weisen 2 bis 10 Dodekapeptide auf, die jeweils durch Spacer von 0 bis 4 Aminosäuren Länge voneinander getrennt sind. Diese Spacer können z. B. aus den Aminosäuren Gly, Ala und Ser bestehen.

Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind beispielsweise aus dem "Lexikon der Biochemie", Spektrum Akademischer Verlag, Berlin, 1999, Band 1, S. 267-271 und Band 2, S. 227-229, bekannt.

Die vorliegende Erfindung umfaßt auch Derivate des erfindungsgemäßen Peptids. Unter Derivaten werden im Sinne der vorliegenden Anmeldung solche Peptide verstanden, deren reine Aminosäurekette chemisch modifiziert worden ist. Solche Derivatisierungen können beispielsweise biologisch im Zusammenhang mit der Biosynthese durch den Wirtsorganismus erfolgen. Hierfür können molekularbiologische Methoden eingesetzt werden. Sie können aber auch chemisch durchgeführt werden, etwa durch die chemische Umwandlung einer Seitenkette einer Aminosäure oder durch kovalente Bindung einer anderen Verbindung an das Peptid. Bei solch einer Verbindung kann es sich beispielsweise auch um andere Peptide oder Proteine handeln, die beispielsweise über bifunktionelle chemische Verbindungen an erfindungsgemäße Peptide gebunden werden. Ebenso ist unter Derivatisierung die kovalente Bindung an einen makromolekularen Träger zu verstehen.

Unter Vektoren werden im Sinne der vorliegenden Erfindung aus Nukleinsäuren bestehende Elemente verstanden, die als kennzeichnenden Nukleinsäurebereich ein interessierendes Gen enthalten. Sie vermögen dieses in einer Spezies oder einer Zellinie über mehrere Generationen oder Zellteilungen hinweg als vom übrigen Genom unabhängig replizierendes, stabiles genetisches Element zu etablieren. Vektoren sind insbesondere bei der Verwendung in Bakterien spezielle Plasmide, also zirkulare genetische Elemente. Man unterscheidet in der Gentechnik einerseits zwischen solchen Vektoren, die der Lagerung und somit gewissermaßen auch der gentechnischen Arbeit dienen, den sogenannten Klonierungsvektoren, und andererseits denen, die die Funktion erfüllen, das interessierende Gen in der Wirtszelle zu realisieren, das heißt, die Expression des betreffenden Peptids zu ermöglichen. Diese Vektoren werden als Expressionsvektoren bezeichnet.

Die für ein erfindungsgemäßes Peptid, insbesondere Dodekapeptid oder ein Multimer eines solchen Peptides kodierende Nukleinsäure wird geeigneterweise in einen Vektor kloniert. Die molekularbiologische Dimension der Erfindung besteht somit in Vektoren mit den Genen für die entsprechenden Peptide. Dazu können beispielsweise solche gehören, die sich von bakteriellen Plasmiden, von Viren oder von Bacteriophagen ableiten, oder überwiegend synthetische Vektoren oder Plasmide mit Elementen verschiedenster Herkunft. Mit den weiteren jeweils vorhandenen genetischen Elementen vermögen Vektoren, sich in den betreffenden Wirtszellen über mehrere Generationen hinweg als stabile Einheiten zu etablieren. Es ist dabei im Sinne der Erfindung unerheblich, ob sie sich extrachomosomal als eigene Einheiten etablieren oder in ein Chromosom integrieren. Welches der zahlreichen aus dem Stand der Technik bekannten Systeme gewählt wird, hängt vom Einzelfall ab. Ausschlaggebend können beispielsweise die erreichbare Kopienzahl, die zur Verfügung stehenden Selektionssysteme, darunter vor allem Antibiotikaresistenzen, oder die Kultivierbarkeit der zur Aufnahme der Vektoren befähigten Wirtszellen sein.

Die Vektoren bilden geeignete Ausgangspunkte für molekularbiologische und biochemische Untersuchungen des betreffenden Gens oder zugehörigen Peptids und für erfindungsgemäße Weiterentwicklungen und letztlich für die Amplifikation und Produktion erfindungsgemäßer Peptide. Sie stellen weitere Ausführungsformen der vorliegenden Erfindung dar.

Bevorzugte Ausführungsformen der vorliegenden Erfindung sind Klonierungsvektoren. Diese eignen sich neben der Lagerung, der biologischen Amplifikation oder der Selektion des interessierenden Gens für die Charakterisierung des betreffenden Gens, etwa über das Erstellen einer Restriktionskarte oder die Sequenzierung. Klonierungsvektoren sind auch deshalb bevorzugte Ausführungsformen der vorliegenden Erfindung, weil sie eine transportierbare und lagerfähige Form der beanspruchten DNA darstellen. Sie sind auch bevorzugte Ausgangspunkte für molekularbiologische Techniken, die nicht an Zellen gebunden sind, wie beispielsweise die Polymerasekettenreaktion.

Expressionsvektoren sind chemisch den Klonierungsvektoren ähnlich, unterscheiden sich aber in jenen Teilsequenzen, die sie dazu befähigen, in den für die Produktion von Peptiden optimierten Wirtsorganismen zu replizieren und dort das enthaltene Gen zur Expression zu bringen. Bevorzugte Ausführungsformen sind Expressionsvektoren, die selbst die zur Expression notwendigen genetischen Elemente tragen. Die Expression wird beispielsweise von Promotoren beeinflußt, welche die Transkription des Gens regulieren. So kann die Expression durch den natürlichen, ursprünglich vor diesem Gen lokalisierten Promotor erfolgen, aber auch nach gentechnischer Fusion sowohl durch einen auf dem Expressionsvektor bereitgestellten Promotor der Wirtszelle als auch durch einen modifizierten oder einen völlig anderen Promotor eines anderen Organismus.

Bevorzugte Ausführungsformen sind solche Expressionsvektoren, die über Änderungen der Kulturbedingungen oder Zugabe von bestimmten Verbindungen, wie beispielsweise die Zelldichte oder spezielle Faktoren, regulierbar sind.

Ausführungsformen der vorliegenden Erfindung können auch zellfreie Expressionssysteme sein, bei denen die Peptidbiosynthese in vitro nachvollzogen wird. Derartige Expressionssysteme sind im Stand der Technik ebenfalls etabliert.

Die In-vivo-Synthese eines erfindungsgemäßen Peptids, also die durch lebende Zellen, erfordert den Transfer des zugehörigen Gens in eine Wirtszelle, deren sogenannte Transformation. Als Wirtszellen eignen sich prinzipiell alle Organismen, das heißt Prokaryonten, Eukaryonten oder Cyanophyta. Bevorzugt sind solche Wirtszellen, die sich genetisch gut handhaben lassen, was beispielsweise die Transformation mit dem Expressionsvektor und dessen stabile Etablierung angeht, beispielsweise einzellige Pilze oder Bakterien. Zudem zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdpeptide. Häufig müssen aus der Fülle an verschiedenen nach dem Stand der Technik zur Verfügung stehenden Systeme die optimalen Expressionssysteme für den Einzelfall experimentell ermitteln werden. Jedes erfindungsgemäße Peptid kann auf diese Weise aus einer Vielzahl von Wirtsorganismen gewonnen werden.

Bevorzugte Ausführungsformen stellen solche Wirtszellen dar, die aufgrund genetischer Regulationselemente, die beispielsweise auf dem Expressionsvektor zur Verfügung gestellt werden, aber auch von vornherein in diesen Zellen vorhanden sein können, in ihrer Aktivität regulierbar sind. Beispielsweise durch kontrollierte Zugabe von chemischen Verbindungen, die als Aktivatoren dienen, durch Änderung der Kultivierungsbedingungen oder bei Erreichen einer bestimmten Zelldichte können diese zur Expression angeregt werden. Dies ermöglicht eine sehr wirtschaftliche Produktion der interessierenden Peptide.

Bevorzugte Wirtszellen sind prokaryontische oder bakterielle Zellen. Bakterien zeichnen sich gegenüber Eukaryonten in der Regel durch kürzere Generationszeiten und geringere Ansprüche an die Kultivierungsbedingungen aus. Dadurch können kostengünstige Verfahren zur Gewinnung erfindungsgemäßer Peptide etabliert werden. Bei gram-negativen Bakterien, wie beispielsweise E. coli, werden eine Vielzahl von Peptiden in den periplasmatischen Raum sekretiert, also in das Kompartiment zwischen den beiden die Zellen einschließenden Membranen. Dies kann für spezielle Anwendungen vorteilhaft sein. Grampositive Bakterien, wie beispielsweise Bacilli oder Actinomyceten oder andere Vertreter der Actinomycetales, besitzen demgegenüber keine äußere Membran, so daß sekretierte Peptide sogleich in das die Zellen umgebende Nährmedium abgegeben werden, aus welchem sich nach einer anderen bevorzugten Ausführungsform die exprimierten erfindungsgemäßen Peptide direkt aufreinigen lassen.

Eine Variante dieses Versuchsprinzips stellen Expressionssysteme dar, bei denen zusätzliche Gene, beispielsweise solche, die auf anderen Vektoren zur Verfügung gestellt werden, die Produktion erfindungsgemäßer Peptide beeinflussen. Hierbei kann es sich um modifizierende Genprodukte handeln oder um solche, die mit dem erfindungsgemäßen Peptid gemeinsam aufgereinigt werden sollen.

Aufgrund der weitreichenden Erfahrungen, die man beispielsweise hinsichtlich der molekularbiologischen Methoden und der Kultivierbarkeit mit coliformen Bakterien hat, stellen diese bevorzugte Ausführungsformen der vorliegenden Erfindung dar. Besonders bevorzugt sind solche der Gattungen Escherichia coli, insbesondere nichtpathogene, für die biotechnologische Produktion geeignete Stämme.

Repräsentative Vertreter dieser Gattungen sind die K12-Derivate und die B-Stämme von Escherichia coli. Stämme, die sich nach an sich bekannten genetischen und/oder mikrobiologischen Methoden von diesen ableiten lassen, und somit als deren Derivate angesehen werden können, besitzen für genetische und mikrobiologische Arbeiten die größte Bedeutung und werden vorzugsweise zur Entwicklung erfindungsgemäßer Verfahren eingesetzt. Solche Derivate können beispielsweise über Deletions- oder Insertionsmutagenese hinsichtlich ihrer Anforderungen an die Kulturbedingungen verändert sein, andere oder zusätzliche Selektionsmarker aufweisen oder andere oder zusätzliche Peptide exprimieren. Es kann sich insbesondere um solche Derivate handeln, die zusätzlich zu dem erfindungsgemäß hergestellten Peptid weitere wirtschaftlich interessante Peptide exprimieren.

Auch solche Mikroorganismen sind bevorzugt, die dadurch gekennzeichnet sind, daß sie nach Transformation mit einem der oben beschriebenen Vektoren erhalten worden sind. Dabei kann es sich beispielsweise um Klonierungsvektoren handeln, die zur Lagerung und/oder Modifikation in einen beliebigen Bakterienstamm eingebracht worden sind. Solche Schritte sind in der Lagerung und in der Weiterentwicklung betreffender genetischer Elemente allgemein verbreitet. Da aus diesen Mikroorganismen die betreffenden genetischen Elemente in zur Expression geeignete gram-negative Bakterien unmittelbar übertragen werden können, handelt es sich auch bei den vorangegenagenen Transformationsprodukten um Verwirklichungen des betreffenden Erfindungsgegenstands.

Auch eukaryontische Zellen können sich zur Produktion erfindungsgemäßer Peptide eignen. Beispiele dafür sind Pilze wie Actinomyceten oder Hefen wie Saccharomyces oder Kluyveromyces. Dies kann beispielsweise dann besonders vorteilhaft sein, wenn die Peptide im Zusammenhang mit ihrer Synthese spezifische Modifikationen erfahren sollen, die derartige Systeme ermöglichen. Dazu gehören beispielsweise die Bindung niedermolekularer Verbindungen wie Membrananker oder Oligosaccharide.

Die Wirtszellen des erfindungsgemäßen Verfahrens werden in an sich bekannter Weise kultiviert und fermentiert, beispielsweise in diskontinuierlichen oder kontinuierlichen Systemen. Im ersten Fall wird ein geeignetes Nährmedium mit den rekombinanten Bakterienstämmen beimpft und das Produkt nach einem experimentell zu ermittelnden Zeitraum aus dem Medium geerntet. Kontinuierliche Fermentationen zeichnen sich durch Erreichen eines Fließgleichgewichts aus, in dem über einen vergleichsweise langen Zeitraum Zellen telweise absterben aber auch nachwachsen und gleichzeitig Produkt aus dem Medium entnommen werden kann.

Fermentationsverfahren sind an sich aus dem Stand der Technik wohlbekannt und stellen den eigentlichen großtechnischen Produktionsschritt dar; gefolgt von einer geeigneten Aufreinigungsmethode.

Alle Fermentationsverfahren, die auf einem der oben ausgeführten Verfahren zur Herstellung der rekombinanten Peptide beruhen, stellen entsprechend bevorzugte Ausführungsformen dieses Erfindungsgegenstandes dar.

Hierbei müssen die für die eingesetzten Herstellungsverfahren, für die Wirtszellen und/oder die herzustellenden Peptide jeweils optimalen Bedingungen anhand der zuvor optimierten Kulturbedingungen der betreffenden Stämme nach dem Wissen des Fachmanns, beispielsweise hinsichtlich Fermentationsvolumen, Medienzusammensetzung, Sauerstoffversorgung oder Rührergeschwindigkeit experimentell ermittelt werden.

Fermentationsverfahren, die dadurch gekennzeichnet sind, daß die Fermentation über eine Zulaufstrategie durchgeführt wird, kommen ebenfalls in Betracht. Hierbei werden, die Medienbestandteile, die durch die fortlaufende Kultivierung verbraucht werden, zugefüttert; man spricht auch von einer Zufütterungsstrategie. Hierdurch können beträchtliche Steigerungen sowohl in der Zelldichte, als auch in der Biotrockenmasse und/oder vor allem der Aktivität des interessierenden Peptids erreicht werden.

Analog dazu kann die Fermentation auch so gestaltet werden, daß unerwünschte Stoffwechselprodukte herausgefiltert oder durch Zugabe von Puffer oder jeweils passende Gegenionen neutralisiert werden.

Das hergestellte Peptid kann nachträglich aus dem Fermentationsmedium geerntet werden. Dieses Fermentationsverfahren ist gegenüber der Produktaufbereitung aus der Trockenmasse bevorzugt, erfordert jedoch die Zurverfügungstellung geeigneter Sekretionsmarker und Transportsysteme.

Ohne Sekretion ist u.U. die Aufreinigung des Peptids aus der Zellmasse erforderlich, auch dazu sind verschiedene Verfahren bekannt, wie Fällung z.B durch Ammoniumsulfat oder Ethanol, oder die chromatographische Reinigung, wenn erforderlich bis zur Homogenität. Die Mehrzahl der beschriebenen technischen Verfahren dürfte jedoch mit einer angereicherten, stabilisierten Präparation auskommen.

Alle bereits oben ausgeführten Elemente können zu Verfahren kombiniert werden, um erfindungsgemäße Peptide herzustellen. Es ist dabei für jedes erfindungsgemäße Peptid eine Vielzahl von Kombinationsmöglichkeiten an Verfahrensschritten denkbar. Das optimale Verfahren muß für jeden konkreten Einzelfall experimentell ermittelt werden.

Weitere Gegenstände der vorliegenden Erfindung sind für das erfindungsgemäße Peptid kodierende Nukleinsäuren sowie Klonierungsvektoren und Expressionsvektoren, die eine für das erfindungsgemäße Peptid kodierende Nukleinsäure enthalten.

Gegenstände der vorliegenden Erfindung sind ferner pro-oder eukaryotische Zellen, die mit einer für das erfindungsgemäße Peptid kodierenden Nukleinsäure transformiert worden sind.

Das erfindungsgemäße Dodekapeptid kann vorteilhafterweise in Beschichtungsmitteln, Lacken und Anstrichmitteln, Haftvermittlern oder Klebstoffen für oxidische Oberflächen eingesetzt werden. Weitere Gegenstände der vorliegenden Erfindung sind daher Beschichtungsmittel, Lacke und Anstrichmittel, Haftvermittler oder Klebstoffe für oxidische Oberflächen, die das erfindungsgemäße Dodekapeptid enthalten, oder aus diesem bestehen.

Ein weiterer Gegenstand der vorliegenden Erfindung ist außerdem ein mehrschichtiger Verbund oder ein beschichtetes Substrat, enthaltend Verbindungen, die vollständig oder zum Teil aus dem Dodekapeptid als Haftvermittler zwischen mindestens zwei benachbarten Schichten des Verbunds oder zwischen der Beschichtung und dem Substrat aufgebaut sind.

Mehrschichtige Verbunde werden für unterschiedlichste Zwecke, z. B. als Verpackungsmittel (insbesondere Verbundfolien) oder selbstklebende Artikel (mehrschichtiger Verbund aus mindestens Träger und Klebstoffschicht) eingesetzt. Die efindungsgemäßen mehrschichtigen Verbunde enthalten mindestens zwei, vorzugsweise zwei bis fünf Schichten, wobei die einzelnen Schichten eine Dicke von z. B. 0,01 bis 5 mm haben können.

Die einzelnen Schichten weisen oxidische Oberflächen auf und enthalten vorzugsweise Metall oder Metalloxide. Bei den Schichten handelt es sich insbesondere um Metallfolien oder metallisierte Polymerfolien.

Zwischen mindestens zwei benachbarten Schichten des mehrschichtigen Verbunds wird das oben genannte Peptid als Haftvermittler verwendet. Vorzugsweise handelt es sich bei mindestens einer der benachbarten Schichten um eine Schicht aus einem metallisierten natürlichen oder synthetischen Polymer. Als Polymere kommen insbesondere Polykondensate, wie Polyester, Polyaddukte wie Polyurethane, Polyamide, Polycarbonate oder Polyphenylenether oder Polyphenylensulfide oder Polymere, welche durch radikalische oder ionische Polymerisation von ethylenisch ungesättigten Verbindungen erhältlich sind (kurz radikalische Polymere genannt). Derartige radikalische Polymere bestehen vorzugsweise zu mindestens 60 Gew.-%, besonders bevorzugt zu mindestens 80 Gew.-% aus sogenannten Hauptmonomeren, ausgewählt aus C1 bis C20 Alkyl(meth)acrylaten, Vinylestern von bis zu 20 C-Atome enthaltenden Carbonsäuren, Vinylaromaten mit bis zu 20 C-Atomen, ethylenisch ungesättigten Nitrilen, Vinylhalogeniden, Vinylethern von 1 bis 10 C Atome enthaltenden Alkoholen, aliphatischen Kohlenwasserstoffen mit 2 bis 8 C Atomen und ein oder zwei Doppelbindungen, insbesondere Ethylen und Propylen.

Die für die Haftvermittling benötigte Menge des Peptids beträgt im Allgemeinen 0,01 bis 1000 mg (Milligramm/m2 (Quadratmeter), insbesondere 0,01 bis 100 mg/m2 und besonders bevorzugt 0,1 bis 10 mg/m2. Das erfindungsgemäße Dodekapeptid kann auf eine der benachbarten Schichten aufgetragen werden, alternativ kann das Peptid auch auf beide Schichten aufgetragen werden.

Das Peptid liegt vorzugsweise als wässrige Lösung vor; der Peptidgehalt der Lösung beträgt vorzugsweise 0,01 bis 5 Gew.-Teile Peptid auf 100 Gew.-Teile Wasser. Für die erfindungsgemäßen Verwendungen in Beschichtungsmitteln, Haftvermittlern oder Klebstoffen für oxidische Oberflächen wird die Lösung vorzugsweise weiter verdünnt auf eine Konzentration von 1 bis 10000 µg/ml Wasser, insbesondere 10 bis 1000 µg/ml Wasser.

Nach dem Auftragen kann daher zunächst eine Trocknung zur Entfernung des Wassers erfolgen. Danach können die beiden benachbarten Schichten durch übliche Methoden, z. B. durch Kaschierung verbunden werden.

Substrate werden zu unterschiedlichsten Zwecken beschichtet. Genannt seien insbesondere dekorative Überzüge oder Schutzüberzüge (zusammenfassend Lackierungen) oder auch Klebstoffbeschichtungen, wobei der Klebstoff als solcher auf das Substrat aufgebracht werden kann oder z. B. in Form eines selbstklebenden Artikels (Etikett oder Klebeband) aufgeklebt wird. Das Substrat, bzw. die Substratoberfläche, kann aus einem beliebigen Material bestehen. Ebenso kann die Beschichtung bzw. die substratseitige Oberfläche der Beschichtung aus einem beliebigen Material bestehen. Vorzugsweise besteht die Substratoberfläche oder die Beschichtung, bzw. die substratseitige Oberfläche der Beschichtung aus Metall, insbesondere aus Stahl, oder aus metallisierten natürlichen oder synthetischen Polymeren.

Als Polymere kommen insbesondere Polykondensate, wie Polyester, Polyaddukte wie Polyurethane, Polyamide, Polycarbonate oder Polyphenylenether oder Polyphenylensulfide oder Polymere, welche durch radikalische oder ionische Polymerisation von ethylenisch ungesättigten Verbindungen erhältlich sind (kurz radikalische Polymere genannt). Zum Aufbau der radikalischen Polymeren und ihren Gehalt an Hauptmonomeren gilt das oben gesagte.

Die für die Haftvermittlung benötigte Menge des Peptids entspricht der oben angegebenen Menge. Das Peptid kann auf die Substratoberfläche, auf die substratseitige Oberfläche der Beschichtung oder auf beide aufgetragen werden. Das Peptid liegt vorzugsweise als wässrige Lösung vor; der Gehalt der Lösung ist wie oben angegeben. Nach dem Auftragen kann daher zunächst eine Trocknung zur Entfernung des Wassers erfolgen.

Die Beschichtung kann nach üblichen Methoden auf das Substrat aufgebracht werden, Folien oder mehrschichtige Verbunde können z. B. aufkaschiert werden. Insbesondere kann die Beschichtung durch Aufbringen einer Polymerdispersion, Polymerlösung oder eines lösemittelfreien Polymeren auf die mit dem Haftvermittler versehene substratseitige Oberfläche und anschließende Verfilmung und/oder thermische oder photochemische Härtung hergestellt werden. Insbesondere liegt das Polymer dazu in Form einer wässrigen Dispersion oder Lösung vor, besonders bevorzugt handelt es sich um eine wässrige Dispersion eines Emulsionspolymerisats, vorzugsweise eines oben aufgeführten radikalischen Polymeren. Nach dem Auftragen des Polymeren erfolgt dann gegebenenfalls eine Trocknung.

Die erfindungsgemäßen mehrschichtigen Verbunde und beschichteten Substrate haben eine deutlich erhöhte Festigkeit. Die Beschichtung haftet durch die Verwendung des Peptids als Haftvermittler stärker auf dem Substrat und die einzelnen Schichten des mehrschichtigen Verbunds haften besser aneinander.

In einer ganz besonders bevorzugten Ausführungsform der Erfindung handelt es sich bei dem Substrat um ein Metall. Es kann sich hierbei prinzipiell um beliebige Metalle handeln. Beispiele umfassen Eisen, Stahl, Zink, Zinn, Aluminium, Kupfer bzw. Legierungen dieser Metalle untereinander sowie mit anderen Metallen handeln. Es kann sich insbesondere um Stahl, mit Zink-, Zinklegierungen, Aluminium- oder Aluminiumlegierungen beschichteten Stahl oder um Aluminium- bzw. Aluminiumlegierungen handeln. Besonders bevorzugt sind Zink- bzw. Zinklegierungen bzw. damit Substrate wie beispielsweise verzinkter Stahl.

Zn- oder Al-Legierungen sind dem Fachmann bekannt. Je nach dem gewünschten Anwendungszweck wählt der Fachmann Art und Menge von Legierungsbestandteilen aus. Typische Bestandteile von Zink-Legierungen umfassen insbesondere Al, Pb, Si, Mg, Sn, Cu oder Cd. Typische Bestandteile von Aluminium-Legierungen umfassen insbesondere Mg, Mn, Si, Zn, Cr, Zr, Cu oder Ti. Es kann sich auch um Al/Zn-Legierungen handeln, bei denen Al- und Zn in annähernd gleicher Menge vorhanden sind. Mit derartigen Legierungen beschichteter Stahl ist kommerziell erhältlich.

Die Metalle können in beliebiger Form vorliegen, bevorzugt sind aber Metallfolien, Metallbänder oder Metallbleche. Bei dem Metall kann es sich auch um ein Verbundmaterial mit einer metallischen Oberfläche handeln. Beispielsweise kann es sich um einen Verbund aus einer Polymerfolie und einem Metall handeln.

Die oxidischen, vorzugsweise metallischen Oberflächen werden mit dem erfindungsgemäßen Peptid als Haftvermittler beschichtet. Dies kann mit einer wässrigen Lösung des Peptids erfolgen. Einzelheiten zur Beschichtung wurden bereits vorstehend erwähnt.

Bei der Beschichtung kann es sich insbesondere um typische Lacke bzw. Lacksysteme zur Beschichtung von metallischen Oberflächen handeln. Es kann sich hierbei um thermisch, fotochemisch oder nach anderen Mechanismen härtende Lacke handeln.

Typische Lacke zur Beschichtung von Metalloberflächen umfassen mindestens ein Bindemittel sowie vernetzbare Komponenten. Bei den vernetzbaren Komponenten kann es sich um Vernetzer handeln, welche zusätzlich zu einem Bindemittel eingesetzt werden oder es kann sich hierbei um vernetzbare Gruppen handeln, welche mit dem Bindemittel verbunden sind. Selbstverständlich kann auch das Bindemittel vernetzbare Gruppen aufweisen und zusätzlich ein Vernetzer eingesetzt werden. Hierbei sind verschiedene Kombinationsmöglichkeiten denkbar. Beispielsweise können Bindemittel und Vernetzer getrennt voneinander eingesetzt werden. Das Bindemittel umfasst dann reaktive funktionelle Gruppen, die mit komplementären, reaktiven funktionellen Gruppen in den Vernetzern reagieren können. Alternativ kann es sich auch um selbstvernetzende Bindemittel handeln, die reaktive funktionelle Gruppen umfassen, die mit Gruppen ihrer Art ("mit sich selbst") oder mit komplementären, reaktiven funktionellen Gruppen am selben Polymer Vernetzungsreaktionen eingehen können. Möglich ist auch, dass ausschließlich die Vernetzer miteinander reagieren.

Beispiele geeigneter Bindemittel umfassen (Meth)acrylat(co)polymerisate, partiell verseifte Polyvinylester, Polyester, Alkydharze, Polylactone, Polycarbonate, Polyether, Epoxidharz-Amin-Addukte, Polyharnstoffe, Polyamide, Polyimide oder Polyurethane. Selbstverständlich können auch Mischungen verschiedener Polymerer eingesetzt werden, vorausgesetzt es treten durch die Mischung keine unerwünschten Effekte auf. Die vernetzenden Komponenten können thermisch vernetzende Gruppen oder fotochemisch vernetzende Gruppen aufweisen. Geeignete thermische Vernetzer sind beispielsweise Vernetzer auf Basis von Epoxiden, von Melamin oder von blockierten Isocyanaten. Geeignete Vernetzer zur photochemischen Vernetzung sind insbesondere Verbindungen mit mehreren ethylenisch ungesättigten Gruppen, insbesondere di- oder polyfunktionelle Acrylate.

Durch das erfindungsgemäße Peptid wird die Haftung des Lackes auf dem Substrat vorteilhaft verbessert. Weiterhin wird in Korrosionsschutztests auch eine verbesserte Beständigkeit gegen Unterwanderung der Lackschicht erreicht.

Ein weiterer Gegenstand der vorliegenden Erfindung ist daher die Verwendung von dem erfindungsgemäßen Peptid als Bestandteil von Beschichtungsmitteln, Lacken und Anstrichmitteln, Haftvermittlern oder Klebstoffen für oxidische Oberflächen sowie von mehrschichtigen Verbunden oder beschichteten Substraten.

Das folgende Beispiel erläutert die Erfindung, ohne sie jedoch darauf einzuschränken:

### Beispiel 1: Quantifizierung von Peptiden auf (Stahl-) Oberflächen

Mit der im folgenden beschriebenen Methode wird illustriert, wie eine Quantifizierung von an (Stahl-) Oberflächen adhärierten Peptiden durchgeführt werden kann.

Damit ist es möglich, über die visuelle Bewertung hinaus ein objektiv messbares Ergebnis bezüglich der Adhäsionseigenschaften zu erzeugen. Dies Verfahren ist beispielhaft zu verstehen, weitere chemische Nachweisverfahren können analog geeignet sein. (z.B. Reaktion mit Ninhydrin).

Mit der beschriebenen Methode können die in Tabelle 3 genannten Peptide diskriminiert werden, neben guten (P6, P7) und mittelmäßigen (P13, P15) können schlecht bis gar nicht bindende Peptide (10, 17) unterschieden werden, wie der in Figur 1 gezeigten Grafik zu entnehmen ist.

### Auftrag der Peptide:

2x 10 µl Peptidlösung (2 mg/ml gelöst in 1xPBS pH 7,4 v. Gibco) werden auf ein HDG-Stahlplättchen (rund, Durchmesser 2,3 cm) aufgetragen. Die ersten 10 µl werden ca. 15' getrocknet, die zweiten 10 µl werden anschließend auf die gleiche Stelle getropft und für 30' getrocknet. Trocknung bei 30°C im Hybridisierungsofen HB-1000 von UVP in Gegenwart von 200 ml Wasser.

### Waschen:

Die Plättchen werden zum Waschen in eine Kristallisierschale (Durchmesser 19cm) mit 1 Liter 1xPBS pH 7,4 gelegt und für 10' bei RT leicht geschüttelt (60 rpm, Certomat U v. B. Braun). Anschließend werden die Plättchen bei 30°C getrocknet.

### Färben:

Die Plättchen werden in eine 6-well Schale überführt. Es werden 150 µl Coomassie - Färbelösung auf das Plättchen pipettiert. Nach 30 sec werden vorsichtig (am Rand) 6ml 1xPBS pH 7,4 Puffer zum Abspülen der Färbelösung hinzugegeben. Danach wird das Plättchen 2x in 6 ml 1xPBS pH 7,4 überführt, um restliche Färbelösung zu entfernen.
Anschließend wird das Plättchen kurz bei 30°C getrocknet.
(Coomassie-Färbelösung.: 2 g Coomassie brilliant blue R-250, 0,5 g Coomassie brilliant blue G-250, 50 ml Methanol, 425 ml Ethanol, 100 ml Eisessig, ad 1000 ml Aqua dest.)

### Ablösen:

Es werden 20 µl DMSO/CH3COOH-Lsg. (v/v 9/1) auf den gefärbten Spot pipettiert. Man lässt die Extraktionslösung 5' bei RT einwirken, anschließend wird die Flüssigkeit unter auf- und ab pipettieren aufgesaugt, bis der Spot vollständig vom Plättchen entfernt ist. Der Überstand wird in ein Reaktionsgefäß überführt.

### Messung:

Die Messung der Farbintensität erfolgt im Nanodrop 1000 Spectrophotometer. (Program UV VIS). Dazu werden 2 µl Probe aufgetragen und bei 602 nm gemessen.

### Tabellen und Figuren:

**Tabelle 1:**

| Position: | 1 | 2 | 3 | 4 | 5 | 6 | 7 | 8 | 9 | 10 | 11 | 12 |
|---|---|---|---|---|---|---|---|---|---|---|---|---|
| Peptid K1 (nicht erfindungsgemäß) | X | K | R | R | P | G | X | X | V | E | X | X |
| Peptid K2 (nicht erfindungsgemäß) | H | N | T | I | R | I | L | T | I | K | L | X |
| Peptid K3 (nicht erfindungsgemäß) | R | H | S | S | T | L | R | Y | R | P | H | P |
| Peptid 8 (nicht erfindungsgemäß) | R | S | I | V | T | F | S | L | R | Q | N | R |
| Peptid 9 (nicht erfindungsgemäß) | Q | R | N | L | I | K | S | T | C | R | K | I |

| | | | | | | | | | | | | |
|---|---|---|---|---|---|---|---|---|---|---|---|---|
| X: jegliche Aminosäure | | | | | | | | | | | | |

**Tabelle 2:**

| Peptid | AA-Sequenz | | | | | | | | | | | | Klebeassay |
|---|---|---|---|---|---|---|---|---|---|---|---|---|---|
| | | | | | | | | | | | | | HDG-Edelstahl |
| Nr. | 1 | 2 | 3 | 4 | 5 | 6 | 7 | 8 | 9 | 10 | 11 | 12 | Quantitative Auswertung Coomassie (abs 602nm) |
| 6 (nicht erfindungsgemäß) | S | R | A | R | L | F | V | V | T | Y | H | K | 0,728 |
| 7 | H | M | I | S | T | M | N | A | A | S | R | R | 0,549 |
| 13 (nicht erfindungsgemäß) | R | N | T | I | R | I | R | T | I | K | H | P | 0,348 |
| 15 (nicht erfindungsgemäß) | R | H | S | S | T | L | R | Y | R | P | L | P | 0,369 |
| 10 (nicht erfindungsgemäß) | R | Q | L | Q | R | L | M | K | S | V | N | S | 0,229 |
| 17 (nicht erfindungsgemäß) | Q | Q | S | R | H | I | L | N | R | K | K | P | 0,186 |

**Tabelle 3:**

| Für erfindungsgemäße Peptide kodierende Nukleotidsequenzen. | |
|---|---|
| Peptid | Sequenz |
| Peptid 6 (nicht erfindungsgemäß) | tccagggcacgactatttgtagtcacatatcacaaa |
| Peptid 7 | cacatgatatctacaatgaacgcggcgagccggcga |
| Peptid 8 (nicht erfindungsgemäß) | agaagcatagtaacctttagtttaagacaaaatcgc |
| Peptid 9 (nicht erfindungsgemäß) | caacgtaatttgattaaatcgacgtgccggaaaatc |
| Peptid 10 (nicht erfindungsgemäß) | cgccagctccagaggctgatgaaatcagtaaattcg |
| Peptid 13 (nicht erfindungsgemäß) | cgcaacactattcgtatacggactataaaacatccg |
| Peptid 15 (nicht erfindungsgemäß) | cgacacagtagtactctgaggtataggccacttccg |
| Peptid 17 (nicht erfindungsgemäß) | caacaaagtcgtcatatactgaatagaaaaaaaccg |

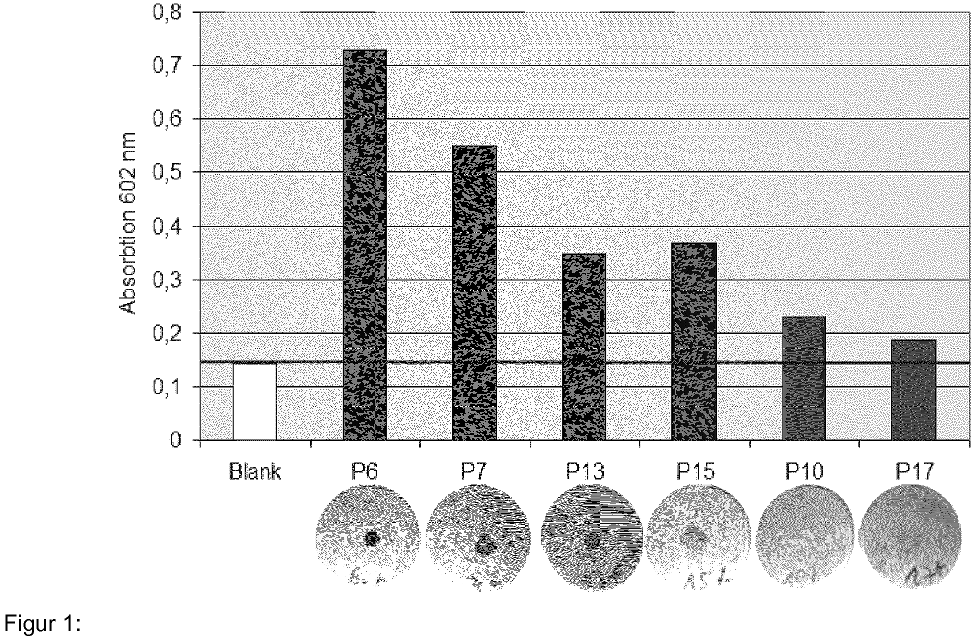

## Patentansprüche

1. Dodekapeptid, **dadurch gekennzeichnet, dass** das Dodekapeptid die Aminosäuresequenz HMISTMNAASRR (SEQ ID NO:10) aufweist.

2. Nukleinsäure, die für das Peptid nach Anspruch 1 kodiert, insbesondere eine solche, die der Nukleinsäuresequenz gemäß SEQ ID NO:2 entspricht.

3. Vektoren, insbesondere Klonierungsvektoren und Expressionsvektoren, die die für ein Dodekapeptid kodierende Nukleinsäure nach Anspruch 2 enthalten.

4. Pro- oder eukaryotische Zellen, die mit der für ein Dodekapeptid kodierenden Nukleinsäure nach Anspruch 2 transformiert worden sind.

5. Beschichtungsmittel, Lacke und Anstrichmittel, Haftvermittler oder Klebstoffe für oxidische Oberflächen, enthaltend das Peptid nach Anspruch 1.

6. Mehrschichtiger Verbund oder beschichtetes Substrat, enthaltend Verbindungen, die vollständig oder zum Teil aus dem Peptid nach Anspruch 1 als Haftvermittler zwischen mindestens zwei benachbarten Schichten des Verbunds oder zwischen der Beschichtung und dem Substrat aufgebaut sind.

7. Verwendung des Peptids nach Anspruch 1 als Bestandteil von Beschichtungsmitteln, Lacken und Anstrichmitteln, Haftvermittlern oder Klebstoffen für oxidische Oberflächen sowie von mehrschichtigen Verbunden oder beschichteten Substraten.

## Claims

1. A dodecapeptide, **characterized in that** the dodecapeptide has the amino acid sequence HMISTMNAASRR (SEQ ID NO: 10).

2. A nucleic acid that codes for the peptide according to claim 1, in particular a nucleic acid corresponding to the nucleic acid sequence according to SEQ ID NO: 2.

3. Vectors, in particular cloning vectors and expression vectors, which contain the nucleic acid according to claim 2 that codes for a dodecapeptide.

4. Prokaryotic or eukaryotic cells that have been transformed by means of the nucleic acid according to claim 2 that codes for a dodecapeptide.

5. Coating agents, varnishes and paints, adhesion promoters or adhesives for oxidic surfaces, containing the peptide according to claim 1.

6. A multilayer composite or coated substrate containing compounds that are completely or partially composed of the peptide according to claim 1 as an adhesion promoter between at least two adjacent layers of the composite or between the coating and the substrate.

7. The use of the peptide according to claim 1 as a component of coating agents, varnishes and paints, adhesion promoters or adhesives for oxidic surfaces and as a component of multilayer composites or coated substrates.

## Revendications

1. Dodécapeptide, **caractérisé en ce que** le dodécapeptide comporte la séquence d'acides aminés HMISTMNAASRR (SEQ ID NO:10).

2. Acide nucléique codant le peptide selon la revendication 1, en particulier un acide nucléique qui correspond à la séquence d'acide nucléique selon SEQ ID NO:2

3. Vecteurs, notamment vecteurs de clonage et vecteurs d'expression, qui contiennent l'acide nucléique codant une dodécapeptide selon la revendication 2.

4. Cellules procaryotes ou eucaryotes qui ont été transformées avec l'acide nucléique codant une dodécapeptide selon la revendication 2.

5. Agent de revêtement, peinture et enduit, agent adhésif ou colle pour surfaces d'oxyde, contenant le peptide selon la revendication 1.

6. Composite multicouche ou un substrat revêtu, comprenant des composés qui sont produits en totalité ou en partie à partir du peptide selon la revendication 1 en tant qu'agent adhésif entre au moins deux couches adjacentes du composite ou entre le revêtement et le substrat.

7. Utilisation du peptide selon la revendication 1 comme composant d'agents de revêtement de surface, de peintures, d'agents adhésifs ou de colles pour surfaces d'oxyde, ainsi que de composites multicouches ou de substrats revêtus.
